# EUROPÄISCHE PATENTSCHRIFT

(11) **EP 2 402 434 B1**
(45) Veröffentlichungstag und Bekanntmachung des Hinweises auf die Patenterteilung: **09.01.2013**
(21) Anmeldenummer: 11005393.1
(22) Anmeldetag: 01.07.2011
(51) Int. Cl.: C12N 5/00

(54) **Verfahren zur Herstellung eines für Knorpelzellen geeigneten Trägers**
Method for producing a scaffold suitable for cartilage cells
Procédé de fabrication d'un support adapté à des cellules de cartilage

(30) Priorität: 02.07.2010 DE 102010025934
(43) Veröffentlichungstag der Anmeldung: 04.01.2012
(73) Patentinhaber: Coastal Research & Management GbR Vertretungsberechtiger Gesellschafter, 24159 Kiel (DE)
(72) Erfinder: Erdmann, Silke, Dr., 22301 Hamburg (DE); Sewing, Judith, 23564 Lübeck (DE); Notbohm, Holger, Prof. Dr., 23627 Groß Grönau (DE)
(74) Vertreter: Lobemeier, Martin Landolf

(56) Entgegenhaltungen:
- WO-A1-2008/023025
- SONG E ET AL: "Collagen scaffolds derived from a marine source and their biocompatibility", BIOMATERIALS, ELSEVIER SCIENCE PUBLISHERS BV., BARKING, GB, Bd. 27, Nr. 15, 1. Mai 2006 (2006-05-01), Seiten 2951-2961, XP025097265, ISSN: 0142-9612, DOI: 10.1016/J.BIOMATERIALS.2006.01.015 [gefunden am 2006-05-01]
- VAGO RAZI: "Beyond the skeleton: Cnidarian biomaterials as bioactive extracellular microenvironments for tissue engineering.", ORGANOGENESIS JAN 2008 LNKD- PUBMED:19279710, Bd. 4, Nr. 1, Januar 2008 (2008-01), Seiten 18-22, XP002662601, ISSN: 1547-6278

## Beschreibung

Die Erfindung betrifft ein Verfahren zur Herstellung eines Trägers für Knorpelzellen.

Kollagene erfüllen wichtige mechanische Funktionen im gesamten Körper, insbesondere im Bindegewebe. Darüber hinaus üben Kollagene wichtige Funktionen in der direkten Umgebung von Zellen aus und sind daher ideal für den Einsatz als Biomaterial für das Tissue Engineering geeignet.

Insbesondere werden Schädigungen des Kollagen Typ II aufweisenden Gelenkknorpels *in vivo* häufig nur mit einem Kollagen-Typ-I enthaltenden Mischgewebe repariert, sodass Patienten mit Knorpelverletzungen oftmals dauerhaft unter der Schädigung des Gelenkknorpels zu leiden haben.

Daher ist man bei derartigen Verletzungen darauf angewiesen, verletztes Knorpelgewebe funktionsgerecht zu erneuern, beispielsweise durch direktes Einbringen von Kollagen in die geschädigten Bereiche oder als Strukturgerüst (Scaffold) zur Kultivierung von Zellen im Bereich des Tissue Engineering.

Die formgebenden Eigenschaften von Kollagen werden darüber hinaus auch zu kosmetischen Zwecken, z.B. zur Faltenunterspritzung, oder zur Herstellung resorbierbarer (Naht-) Materialien genutzt.

Üblicherweise ist man für diese medizinischen und kosmetischen Zwecke auf xenogenes Kollagen angewiesen, wobei hauptsächlich auf Rinderkollagen zurückgegriffen wird.

Nachteilig an der Verwendung von Rinderkollagen ist jedoch, dass Krankheiten, z.B. BSE und TSE, auf den Menschen übertragen werden können und ein hoher Aufwand betrieben werden muss, um dieses zu vermeiden.

Daher wurde bereits nach anderen Quellen für Kollagene gesucht, die für die oben genannten Zwecke geeignet sind.

Beispielsweise wurden Kollagene aus mehreren marinen Organismen, etwa aus marinen Schwämmen (Porifera; siehe WO 2008/023025 A1) oder Quallen (E Song et al. Collagen scaffolds derived from a marine source and their biocompatibility. Biomaterials 27 (2006) 2951-2961), isoliert. Der Vorteil bei der Verwendung von marinen Organismen als Quelle für Kollagene liegt einerseits in der leichten Verfügbarkeit des Ausgangsmaterials sowie andererseits in der in Bezug auf BSE- oder TSE-Krankheitserreger risikolosen Anwendung.

Bei den Kollagenen von wirbellosen Tieren zur medizinischen oder kosmetischen Verwendung besteht jedoch die Möglichkeit einer Bioinkompatibilität des Kollagens mit dem menschlichen Organismus aufgrund einer möglichen Antigenität und/oder einer geringeren Thermostabilität des marinen Kollagens, die auf die Anpassung der marinen Organismen auf deren relativ kalten Lebensraum zurückzuführen ist.

Aufgabe der vorliegenden Erfindung ist es daher, ein Verfahren zur Herstellung eines für das Tissue Engineering geeigneten Trägers, insbesondere für Knorpelzellen, bereitzustellen, das eine verbesserte Geweberegeneration, insbesondere Knorpelregeneration, erlaubt, wobei das Risiko einer TSE- oder BSE-Übertragung ausgeschlossen sein sollte.

Diese Aufgabe wird durch das Verfahren mit den Merkmalen von Anspruch 1 gelöst. Die Unteransprüche geben vorteilhafte Ausgestaltungen der Erfindung wieder.

Mit dem Verfahren der vorliegenden Erfindung ist es möglich, ein aus Kollagen bestehenden Träger für das Knorpel-Tissue-Engineering zu schaffen und in der Folge auf diesem hyalinen Knorpel zu entwickeln, wobei als Ausgangsmaterial auf aus einem Nesseltier gewonnenes Kollagen zurückgegriffen wird.

Qualitative Analysen dieser als Scaffolds bezeichneten Träger zeigen eine Zelladhäsion von Knorpelzellen (Chondrozyten) an die Matrix, Zellproliferation und Zellvitalität sowie neue Matrixablagerung. Histologische Analysen ergaben zudem, dass die neu synthetisierte Matrix aus Proteoglykanen und Kollagenen besteht - also dem Hauptbestandteil hyalinen Gelenkknorpels.

Quantitative Analysen auf Proteinebene zeigen eine Kollagenverhältnis des Chondrozytenspezifischen Markers für Kollagen Typ II zum Dedifferenzierungsmarker der Chondrozyten, Kollagen Typ I, von etwa 50:1. Auf dieser Grundlage gleicht der *in vitro* hergestellte mit Chondrozyten besiedelte marine Träger einem hyalin-ähnlichen Knorpelgewebe mit einer mechanisch funktionellen Kollagen Typ-II-Vernetzung.

Da Chondrozyten - wie oben genannt - Knorpelschäden *in vivo* in der Regel nicht erfolgreich reparieren (können), resultieren Knorpelreparaturgewebe oft im Aufbau in einer Kollagen-Typ-I-Matrix, die allerdings den mechanischen Belastungen der Gelenke nicht standhält. Hingegen zeigt das aus marinen Organismen hergestellte Gerüst einen viel versprechenden Ansatz zur funktionalen Reparatur von Gelenkknorpelschädigungen.

Da auch die Immunreaktivität des aus *Cnidaria* gewonnen Kollagens unproblematisch erscheint, kann die neue Kollagenquelle als Alternative zum Rinderkollagen, das grundsätzlich mit einem Kontaminationsrisiko von BSE und TSE verbunden ist, eingesetzt werden.

Das Kollagen wird aus Nesseltieren (Cnidaria), besonders vorteilhaft aus Rhopilema spec., insbesondere Rhopilema esculenta oder Rhopilema hispidum, gewonnen, wobei auch andere Cnidaria in Frage kommen. Bevorzugt wird die Meduse der Tiere als Ausgangsmaterial verwendet.

Beispielsweise wurden gepökelte Rhopilema esculenta Individuen in etwa 5x5 cm große Stücke geschnitten, zunächst mit Leitungswasser grob gespült und anschließend mit MilliQ Wasser gespült bzw. gerührt bis die Salinität im Spülwasser nach wenigstens einer Stunde kleiner oder gleich 0,1 % betrug.

Das gespülte Material wurde in kleine Stücke zerhäckselt und darauf im fünffachen Volumen (w/v) des Ausgangsmaterials für wenigstens 20 min in 0,05 % Essigsäure äquilibriert und der Überstand abgegossen. Anschließend wurde das Material im sechsfachen Volumen 0,05 % Essigsäure mit 10 mg Pepsin (Biozym Hamburg) pro g Ausgangsmaterial aufgenommen.

Das Material wurde bei 4 °C unter Rühren für ungefähr 60 h verdaut, jedenfalls bis keine Klümpchen mehr vorhanden waren.

Die Lösung aus Pepsin enthaltender Essigsäure und verdautem Ausgangsmaterial wurde für 30 min bei 17.000 g zentrifugiert und das daraus resultierende Pellet verworfen. Der Überstand wurde zur Inaktivierung des Pepsins mit NaOH (-Plätzchen) auf einen pH-Wert von 6,5 bis 7 eingestellt (wenn die Lösung nicht bereits vor Zentrifugation neutralisiert wurde).

Darauf wurde das Kollagen zunächst mittels Hinzugeben von NaH₂PO₄ oder Na₂HPO₄ bis zu einer Endkonzentration von 0,2 M gefällt und in Lösung verbleibendes Kollagen anschließend mit KCl bei einer Endkonzentration von 17,5 % (w/v) gefällt.

Alternativ wurde eine Fällung im sauren Milieu durch Hinzugeben von NaCl bis zu einer Konzentration von 17,5 % (w/v) und daraufhin erst gelöst verbleibenden Kollagens durch Hinzugeben von NaH₂PO₄ oder Na₂HPO₄ bis zu einer Konzentration von 0,2 M gefällt, wobei die Lösung erst anschließend durch Hinzugeben von NaOH neutralisiert wurde.

Nach Fällung und gegebenenfalls Neutralisierung der Lösung wurde die Lösung bei wenigstens 5.000 g zentrifugiert und die Überstände verworfen. Das gefällte Kollagen konnte darauf hin entnommen und weiterverarbeitet werden.

Insbesondere wurde das Kollagenpellet in 0,05 % Essigsäure resuspendiert und gegen wenigstens das 10-fache Volumen 0,05 % Essigsäure dialysiert, wobei die Membran eine Trenngröße von etwa 12-16 kDa aufwies.

Die dialysierte Kollagenlösung wurde idealerweise gelöst bei -20 °C oder -80 °C oder nach Gefriertrocknung bei 4 °C oder kälter aufbewahrt.

Das gefriergetrocknete Kollagen wurde wiederum als Ausgangsmaterial zur Herstellung der Bioscaffolds verwendet. Insbesondere lassen sich Kollagenfibrillen aus den genannten marinen Organismen herstellen aus einer (hoch) gereinigten marinen Collagen-Präparation (ca. 94%) in gepufferten Lösung im schwach alkalischen pH-Bereich in einem Konzentrationsbereich bis 20 mg/ml, optimalerweise bis 10 mg/ml, wobei die gepufferte Lösung einen (sub)physiologischen Salzgehalt (NaCl/KCl) aufweist.

Das Kollagen wird in der gewünschten Konzentration gelöst und luftblasenfrei in eine Form für die Zellträger (Bioscaffolds) pipettiert. Daraufhin wird diese über Nacht gelagert, um anschließend bei -20 °C eingefroren zu werden. Das Einfrieren erfolgt unter langsamem Abkühlen in einer Styroporbox. Diese langsame Einfrierkinetik hat Einfluss auf die Ausbildung der gleichmäßig feinporigen Subarchitektur der Scaffolds. Auf eine strikt aseptische Arbeitsweise ist selbstverständlich zu achten.

Beispielsweise ist die Abkühl- bzw. Einfrierkinetik etwa linear mit beispielsweise im Durchschnitt -0,05 bis -0,25 °C pro Minute, bevorzugt -0,1 bis -0,2 °C pro Minute.

Methodisch sei darauf hingewiesen, dass das erfindungsgemäße Protokoll für die Herstellung von Invertebraten-Kollagenfibrillen von dem Herstellungsverfahren für Kollagenfibrillen aus Vertebraten deutlich abweicht. Insbesondere ist eine hohe Konzentration von Nesseltierkollagen, bevorzugt 10 bis 20 mg/ml, besonders bevorzugt 18 bis 20 mg/ml und äußerst bevorzugt 20 mg/ml, zur Ausbildung besonders gut für den Knorpelersatz verwendbarer Scaffolds nach der Erfindung notwendig - die hierfür im Verhältnis zu Vertebratenkollagen (verschwenderisch) hohe Menge an Kollagen ist bei Nesseltierkollagen unproblematisch.

Die Kollagenmoleküle aus den marinen Organismen, z.B. Aurelia aurita, Rhopilema hispidum und Rhopilema esculenta, deren Eigenschaften in Tabelle 3 wiedergegeben sind, bilden in Lösung Fibrillen.

Im Vergleich zu den klassischen Kollagenfibrillen des Typ I aus Vertebraten sind die Fibrillen aus den marinen Organismen dünn. Die Dicke der in vitro gezüchteten Fibrillen aus marinen Kollagenen beträgt durchschnittlich 20-30 nm, kann jedoch ein einzelnen Fällen bis zu knapp 60 nm betragen. In dieser Hinsicht besitzt die marine Kollagenfibrille Ähnlichkeit mit den Kollagenfibrillen vom Vertebraten-Typ II.

Da die Quallenkollagene einen natürlichen Schmelzpunkt haben, der unter der menschlichen Körpertemperatur liegt, müssen die Zellträger oder Scaffolds aus marinen Kollagenen für ein praktikables Medizinprodukt fixiert/modifiziert werden, was auf zwei Art und Weisen geschehen kann, nämlich durch physikalische Behandlung durch W-Hitze oder eine chemische Behandlung durch Reaktion mit Carbodiimiden, die zur Steigerung der ThermoStabilität durch die Ausbildung von Quervernetzungen der Kollagenmoleküle beitragen.

Die physikalische Behandlung beruht auf einer radikalischen Reaktion an aromatischen Aminosäuren des Moleküls gepaart mit einer nachfolgenden Dehydrierung des Kollagens, die zur Erhöhung der helix-to-coil-transition-temperature, also der Denaturierungs-Temperatur durch Entfernung von H₂O führt. Kondensationsreaktionen führen zur Bildung von Ester- und Amidbindungen.

Die chemische Vernetzung beruht auf einer chemischen Reaktion an Säuregruppen von Aminosäuren, die zur Ausbildung von Amidbindungen führt.

Das Procedere der Quervernetzung mittels physikalischer Fixierung erfolgt dadurch, dass die lyophylisierten Scaffolds beidseitig für je 1 Stunde auf einem UV-Tisch bei 312 nm bestrahlt

(Firma: Phase, Lübeck), alternativ bei 254 nm für je 60 Minuten entsprechend 106 J pro Scaffold-Seite (Stratalinker 1800; Firma: Stratagene) bestrahlt werden. Anschließend werden die UV-fixierten Scaffolds in einem Heizblock für eine Stunde bei 110 °C Hitzenachbehandelt. Die ganze Prozedur erfolgt unter aseptischen Bedingungen.

Bei der chemischen Fixierung nutzt man die quervemetzenden Eigenschaften einer EDC-Lösung (EDC: 1-Ethyl-3-(3-dimethylaminopropyl)carbodiimid; Zusammensetzung: EDC 33 mM, Ethanol 40 %, MES Lösung pH 5,0 50mM, NHS Lösung 20 mM). Die Scaffolds werden in eine EDC-Gebrauchslösung gesetzt und darin 4 Stunden inkubiert. Daraufhin erfolgt ein mindestens zweitägiger Waschschritt in entweder Wasser oder PBS, versetzt mit Antibiotika und Antimycotika.

Dabei hat die Endkonzentration des EDC einen wesentlichen Einfluss auf die Kompressionssteifigkeit des Scaffolds, die in einem Bereich von etwa 2 kPa bis zu etwa 50 kPa stufenlos eingestellt werden kann, ohne die Porengröße und/oder die Substratkonzentration zu variieren. Das Einstellen der Kompressionssteifigkeit funktioniert insbesondere bei hohen Konzentrationen von Nesseltierkollagen, speziell bei 20 mg/ml Nesseltierkollagen. Daher muss durch Bereitstellen des erfindungsgemäßen Verfahrens nicht auf andere Systeme, wie etwa 2D-Scaffolds oder Agarosesysteme, ausgewichen werden.

Aus der Fibrillenlösung konnte damit ein mechanisch stabiler Zellträger für das Tissue Engineering hergestellt werden. Der Zellträger oder sog. Fibrillen-Scaffolds dient insbesondere der in vitro Züchtung von Knorpelgewebe. Dabei unterscheiden sich diese Fibrillen-Scaffolds in ihren physikalischen Eigenschaften deutlich von den klassisch hergestellten, nicht fibrillären Scaffolds. Zu diesen Eigenschaften gehören: Form- und Größenstabilität in Zellkultur, Steifigkeit, Farbtiefe und Widerstandsfähigkeit gegenüber chemischen Agenzien.

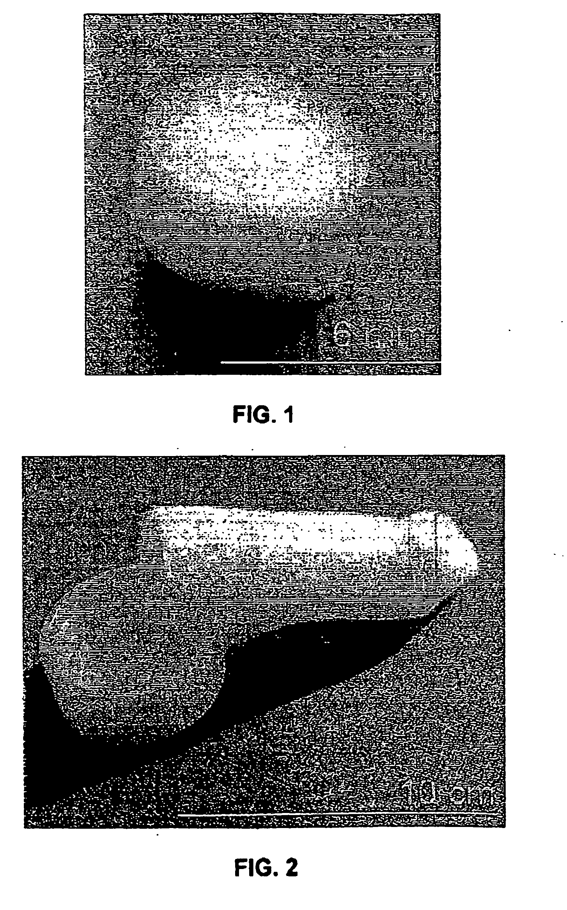

Zwei erfindungsgemäß hergestellte Träger sind in Fig. 1 und Fig. 2 abgebildet. Deren Eigenschaften sind in Tabelle 1 und Tabelle 2 zusammengefasst.

**Tabelle 1**

| | Rhopilema spec. | Aurelia spec. | Methode |
|---|---|---|---|
| | Mikroporöses Schwämmchen | Mikroporöses Schwämmchen | |
| Fixierung | UV-Licht, Hitze | UV-Licht, Hitze | |
| Porengröße | durchschnittlich 100 µm | durchschnittlich 100 µm | Licht-/ Raster-EM-Mikroskopie |
| Gesamttextur | offenporig | offenporig | Licht-/ Raster-EM-Mikroskopie |
| Thermostabilität | a) 37°C | a) 37°C | a) feuchte Hitze |
| | b) 110°C | b) b) 110°C | b) trockene Hitze |
| Chemostabilität | Gut gegenüber | Gut gegenüber | Pinzetten-Test |
| | • Wasser | • Wasser | |
| | • (verdünnten) Säuren | • (verdünnten) Säuren | |
| | • Puffern bis pH 10 | | |
| | mäßig gegenüber | | |
| | • konzentrierten | | |
| | Säuren, | | |
| | Ethanol | | |
| | kaum gegenüber | | |
| | • Natronlauge, | | |
| | pH14 | | |
| Mechano-Stabilität | + | + | Pinzetten-Test/ |
| | + | + | Behrens-Handling-Test |
| Form-Stabilität | bis min. zu 4 Wochen gegeben | bis min. zu 4 Wochen gegeben | unter Zellkulturbedingungen |
| Immunpotential | gering | gering | subkutane Implantation an Mäusen |

**Tabelle 2**

| Eigenschaft | Ergebnis | Methode |
|---|---|---|
| Anhaftung der Zellen an das marine Kollagen | bestätigt; Anhaftung schwächer als die an Vertebraten-Kollagene | Histologie/ Attachment-Assay |
| Lebensfähigkeit der Zellen in marinen Scaffolds | bestätigt; gesichert bis 2 Monate (wahrscheinlich 5) | MTT-/MTS-Test Histologie/ |
| • Zytotoxizität | gering, da Lebensfähigkeit der Zellen über mehrere Monate | Histologie |
| • Zellkompatibilität | "hoch", da Lebensfähigkeit der Zellen über mehrere Monate | Histologie |
| Proliferation | bestätigt; keine exakte Quantifizierung | Histologie/ MTT-/MTS-Test |
| Kollagen-Neu-Synthese | bestätigt für die Kollagen-Typen II und I Ratio: II/I = bis 50 x | Western Blot Analyse/ (Immun)Histologie (= Azan-Färbung) |
| Proteoglykan-Neu-Synthese | bestätigt für sulfatierte Mukosasubstanzen | Histologie (= Alcian-Färbung) |

**Tabelle 3**

| | Rhopilema spec. | Aurelia spec. | Verfahren |
|---|---|---|---|
| Molekulargewicht | 100 kD | 100 kD | SDS-PAGE |
| Alphakettenzusammensetzung | [3 α1], [2 α1, α2] | [α1,α2,α3] | SDS-PAGE |
| Schmelztemperatur | 32,5 °C | 27 °C | |
| Glykosylierung: Glc-Gal-Hyl / Kollagemolekül | 12 kein Gal-Hyl | 12 kein Gal-Hyl | HPLC |
| Quervernetzung | Hoher Anteil an DHLNL, hohe unbekannte Quervernetzung | Hoher Anteil an DHLNL, hohe unbekannte Quervernetzung | Aminosäureanalyse |
| Reinheit | 94% | 98% | Aminosäureanalyse |
| Aminosäurezusammensetzung im Vergleich zu Säugetierkollagen | ca. -30% Prolin, -50% Hydroxyprolin | ca. -30% Prolin, -50% Hydroxyprolin | Aminosäureanalyse |
| Reaktion mit bekannten anti-Kollagen-Antikörpern | keine | keine | Westernblot |
| in vitro Fibrillenbildung | dünne Fibrillen, sehr hohe kritische Konzentration | dünne Fibrillen, sehr hohe kritische Konzentration | Raster-Elektronenmikroskopie |
| ähnlich Vertebratenkollagen | Typ II | Typ II | |

Die Träger eignen sich für das Tissue Engineering zum Besiedeln mit einer Vielzahl unterschiedlicher Zellen, u.a. auch mit Stammzellen. Als besonders vorteilhafte Verwendung der Träger hat sich die Besiedlung der Träger mit Chondrozyten zur Herstellung von als Knorpelgewebeersatz geeigneter Bioscaffolds herausgestellt. Die Kollagen-Neusynthese gemessen an dem von den auf dem Träger kultivierten Chondrozyten neu synthetisierten Kollagen Typ II und neu synthetisiertem Kollagen Typ I beträgt im Verhältnis von Kollagen Typ II zu Kollagen Typ I bis zu 50 : 1 (siehe Tabelle 2), wobei ein Verhältnis von größer 30: 1, mindestens aber größer als 20 : 1 regelmäßig erreicht wird.

Daher bietet das erfindungsgemäße Verfahren eine optimale Grundlage für das Bioengineering von Knorpelersatz.

## Patentansprüche

1. Verfahren zur Herstellung eines Trägers zur Kultivierung von Zellen,
**gekennzeichnet durch** die Schritte
- luftblasenfreies Einfüllen einer höchstens 20 mg Nesseltierkollagen / ml enthaltenden Lösung in eine die äußere Form des Trägers bestimmende Form,
- langsames Abkühlen des in der Form eingefüllten Kollagens auf -20 °C,
- Gefriertrocknen des in der Form eingefüllten Kollagens, und
- Fixieren des gefriergetrockneten Kollagens.

2. Verfahren nach Anspruch 1, **dadurch gekennzeichnet, dass** Fixieren durch Bestrahlen mit UV-Licht erfolgt.

3. Verfahren nach Anspruch 2, **gekennzeichnet durch** Erwärmen des gefriergetrockneten und bestrahlten Kollagens auf 110 °C.

4. Verfahren nach Anspruch 2, **dadurch gekennzeichnet, dass** das Bestrahlen bei 254 nm oder 312 nm erfolgt.

5. Verfahren nach Anspruch 1, **dadurch gekennzeichnet, dass** das Fixieren durch Inkubieren des gefriergetrockneten Kollagens mit einer 1-Ethyl-3-(3-dimethylaminopropyl)carbodiimid enthaltenden Lösung erfolgt.

6. Verfahren nach einem der vorhergehenden Ansprüche, **dadurch gekennzeichnet, dass** das Nesseltier ausgewählt ist aus der Gruppe von Nesseltieren, bestehend aus Rhopilema spec. und Aurelia spec..

7. Verfahren nach Anspruch 6, **dadurch gekennzeichnet, dass** das Kollagen aus Rhopilema esculenta, Rhopilema hispidum oder Aurelia aurita gewonnen wird.

## Claims

1. A method for producing a carrier for cultivating cells,
**characterized by** the following steps
- air-bubble-free filling a solution containing 20 mg cnidarian collagen/ml into a mould determining the outer shape of the carrier ,
- slowly cooling the collagen filled into the mould to -20 °C,
- freeze-drying the collagen filled into the mould, and
- fixating the freeze-dried collagen.

2. The method according to Claim 1, **characterized in that** fixating takes place by irradiating using UV light.

3. The method according to Claim 2, **characterized by** heating the freeze-dried and irradiated collagen to 110°C.

4. The method according to Claim 2, **characterized in that** irradiation takes place at 254 nm or 312 nm.

5. The method according to Claim 1, **characterized in that** fixating takes place by incubating the freeze-dried collagen using a solution containing 1-methyl-3-(3-dimethylaminopropyl)carbodiimide.

6. The method according to one of the preceding claims, **characterized in that** the cnidaria is selected from the group of cnidarians consisting of Rhopilema spec. and Aurelia spec.

7. The method according to Claim 6, **characterized in that** the collagen is produced from Rhopilema esculenta, Rhopilema hispidum or Aurelia aurita.

## Revendications

1. Procédé pour préparer un support pour la culture de cellules,
**Caractérisé par** les étapes
- remplissage sans bulles d'air d'une solution contenant au maximum 20 mg de collagène extrait de cnidaires / ml dans un moule qui détermine la forme extérieure du support,
- lent refroidissement du collagène, versé dans le moule, sur -20 °C,
- lyophilisation du collagène, versé dans le moule, et
- fixation du collagène lyophilisé.

2. Procédé d'après revendication 1, **caractérisé en ce que** la fixation se réalise par exposition à la lumière UV.

3. Procédé d'après revendication 2, **caractérisée par** le fait du chauffage du collagène lyophilisé et exposé à la lumière sur 110 °C.

4. Procédé d'après revendication 2, **caractérisé en ce que** l'exposition aux rayons s'effectue à 254 nm ou 312 nm.

5. Procédé d'après revendication 1, **caractérisé en ce que** la fixation s'effectue par incubation du collagène lyophilisé avec une solution contenant du 1-éthyle-3-(3-dimethylaminopropyl) carbodiimide.

6. Procédé d'après l'une des revendications précédentes, **caractérisé en ce que** le cnidaire est choisi du groupe des cnidaires, consistant en Rhopilema spec. et Aurelia spec.

7. Procédé d'après revendication 6, **caractérisé en ce que** le collagène est extrait de Rhopilema esculenta, Rhopilema hispidum ou Aurelia aurita.
